(19) Europäisches Patentamt
European Patent Office
Office européen des brevets

(11) Veröffentlichungsnummer: **0 100 752**
**A2**

(12) # EUROPÄISCHE PATENTANMELDUNG

(21) Anmeldenummer: **83810344.8**

(22) Anmeldetag: **02.08.83**

(51) Int. Cl.³: **A 61 K 39/205**
**C 12 N 7/08**

(30) Priorität: **04.08.82 CH 4699/82**

(43) Veröffentlichungstag der Anmeldung:
**15.02.84 Patentblatt 84/7**

(84) Benannte Vertragsstaaten:
**AT CH DE FR LI**

(71) Anmelder: **Veterinär-Bakteriologisches Institut der Universität Bern**
**Länggass-Strasse 122**
**CH-3012 Bern(CH)**

(72) Erfinder: **Steck, Franz, Prof. Dr.**
**Eichholzstrasse 121**
**CH-3084 Wabern(CH)**

(74) Vertreter: **Braun, André et al,**
**A. Braun, Braun, Héritier, Eschmann AG Patentanwälte**
**Holbeinstrasse 36-38**
**CH-4051 Basel(CH)**

(54) **Verfahren zur Herstellung eines neuen Tollwut-Impfstoffs, Behälter und Mittel zu dessen Verabreichung und Verfahren zur oralen Immunisierung.**

(57) Der neue Virusstamm SAD produziert auf $BHK_{21}$ wird durch Adaptation eines Tollwutvirus an die Baby Hamster Nierenzellinie $BHK_{21}$ erhalten. Nach Abfüllen in einen für Füchse zerbeissbaren Behälter, der aus einer weichmacherfreien Hart-PVC Schüsselfolie und einer Deckblattfolie aus thermolackiertem Aluminium bestehen kann, wird das Mittel in einem Köder den Füchsen zur oralen Verabreichung dargeboten.

EP 0 100 752 A2

Verfahren zur Herstellung eines neuen Tollwut-Impfstoffs, Behälter und Mittel zu dessen Verabreichung und Verfahren zur oralen Immunisierung

Bekanntlich ist der Fuchs Hauptträger und Verbreiter der Tollwut. Bei der Bekämpfung der Wildtiertollwut muss deshalb eine Herabsetzung der Tollwuthäufigkeit beim Fuchs angestrebt werden. Ganz allgemein gibt es drei Möglichkeiten, eine Infektionskette zum Abbruch zu bringen:

1. natürliche Immunität
2. drastische Bestandesreduktion
3. künstliche Immunisierung

Wie serologische Untersuchungen gezeigt haben, hinterlässt die Tollwut eine fast vollempfängliche Restpopulation, da natürlich erworbene Antikörper nur in einem sehr geringen Prozentsatz festgestellt werden. Deshalb wurde bisher durch Reduktion des Fuchsbestandes dessen Dichte so weit vermindert, dass es zum Abreissen der Infektionskette kam. Unter topographisch günstigen Bedingungen konnte dadurch das Vordringen der Tollwut aufgehalten werden.

Ziel der Erfindung ist es nun, ein Mittel zu finden, das geeignet ist, durch künstliche Immunisierung der Fuchspopulation die Tollwutausbreitung zu verhindern und verseuchte Zonen zu verkleinern bzw. aufzuheben.

Zur Lösung dieser Aufgabe wird ein zur oralen Immunisierung geeignetes Mittel vorgeschlagen, das durch einen in einem erfindungsgemässen Behälter befindlichen erfindungsgemäss geeigneten, gegebenenfalls stabilisierten Impfstoff gekennzeichnet ist. Dieses Mittel wird in einem als Vehikel geeigneten Köder fixiert und im verseuchten Gebiet zum Fressen ausgelegt. Durch Fressen des Köders wird der Behälter aufgebissen und zerkaut und gibt dadurch den Impfstoff in der Maulhöhle frei, so dass dieser über die oropharyngeale Schleimhaut aufgenommen werden kann. Diese Immunisierung führt bei Füchsen in der Praxis zu lebenslänglicher Resistenz gegenüber Tollwutviren.

Als Impfvirus gelangt der attenuierte neue Tollwutvirusstamm SAD produziert auf $BHK_{21}$ zur Anwendung. Zu dessen Herstellung wurde ein Tollwutvirusstamm SAD vom US National Center of Disease Control, Atlanta, USA, verwendet. Der erfindungsgemässe neue Impfstoff wird hergestellt, indem man bei einer Temperatur von 33 bis 37°C, vorzugsweise 33°C, und einem pH-Wert von 6,8 bis 7,4, vorzugsweise 7,4 , Zellen des Virusstammes SAD in gepuffertem Medium, vorzugsweise durch Zugabe einer Bicarbonatlösung, suspendiert und mit einer Zelldichte von 0,5 bis $4 \times 10^5$, vorzugsweise $2 \times 10^5$ Zellen pro ml Medium, vorzugsweise in Rouxflaschen, inokuliert.

Nach Bildung eines vollständigen Zellrasens wird dieser abgelöst, vorzugsweise mit Trypsin-Versenlösung, und gegebenenfalls entsprechend der erhöhten Zellzahl verdünnt und wiederum ausgesät. Die Virusernte aus dem Zellüberstand erfolgt nach ca. 48 bis 72 weiteren Stunden, vorzugsweise 48 Stunden. Unter Einhaltung dieser Bedingungen werden regelmässig Virustiter von $10^{7.0}$ bis $10^{7.5}$ I.E./ml erhalten.

Falls man auf einen Zelltransfer verzichtet, kann und muss initial mit einer höheren Virusdosis inokuliert werden, um entsprechende Virustiter zu erhalten.

In der 12-bis 72-stündigen Zeitspanne, die vom Auslegen des Köders bis zur Aufnahme durch den Fuchs verstreicht, ist der Impfstoff Umwelteinflüssen ausgesetzt. Wärme, Ködersubstanz oder Verpackungsmaterial können die Infektiosität des attenuierten Virus drastisch senken und dabei seine immunisierende Wirkung aufheben. Deshalb wird der Impfstoff vorzugsweise so stabilisiert, dass auch bei erhöhten Temperaturen, z.B. 37°C, innerhalb von z.B. 72 Stunden kein Virustiterverlust grösser als 90% eintritt. Umgekehrt ist es zweckmässig, wenn das verwendete Lebendvirus nach gewisser Zeit spontan inaktiviert wird.

Es hat sich nun gezeigt, dass z.B. durch Zusatz von 5 bis 10% frischem Eigelb oder Eipulver, NTE-Puffer (Tris-Nacl-EDTA, pH 7,4) oder 10% Eigelb plus 8% Serum als Stabilisatoren zum serumfreien Virus eine bedeutend geringere Wärmeinaktivierung eintritt. Dieser Effekt wird in der Regel durch Wegverdünnen des gegebenenfalls vorhandenen Serums noch erhöht.

Durch Ergreifen dieser Massnahmen wird ein maximaler Titerverlust von maximal 1 $\log_{10}$ nach 72 Stunden bei 37°C gemessen.

Funktionell und für die Massenproduktion geeignete Behälter, die den Impfstoff in der Maulhöhle der Füchse freigeben und gleichzeitig vor milieubedingten Titerverlusten schützen, kommen zur Anwendung. Da der Behälter durch den Fuchs zerkaut und aufgebissen werden muss, kommen nur Kunststoffbehälter in Frage. Es hat sich jedoch gezeigt, dass in Kunststoffbehältern Verluste an Infektiosität durch Adsorption oder Inaktivierung Schwierigkeiten bereiten. Auch kam die in Glasbehältern beobachtete stabilisierende Wirkung von z.B. Eigelb in den meisten Kunststoffbehältern nur ungenügend zur Geltung.

Unter einer Vielzahl von Kunststoffbehältern erwiesen sich vorzugsweise Durchdrückpackungen, bestehend aus einer Schüsselfolie und einer Deckblattfolie, als geeignet. Die Schüsselfolie kann z.B. aus einem starren PVC-Film aus weichmacherfreiem Hart-PVC, vorzugsweise 250 µ dick, VC-Gehalt $<1$ ppm und einer Deckblatt-Aluminiumfolie, vorzugsweise 40 µ dick, die auf der Innenseite thermolackiert ist (Vinylbasis 5 bis 6 $g/m^2$), bestehen.

Das erfindungsgemässe Mittel besteht aus einem mit ml, vorzugsweise 1,8 bis 2 ml, gegebenenfalls mit Eigelb stabilisiertem Impfstoff gefüllten erfindungsgemässen Behälter. Die Minimaldosis für die Feldapplikation beträgt vorzugsweise $10^7$ gewebekulturinfektiöse Einheiten.

Das Mittel wird nun in einem als Vehikel geeigneten Köder so fixiert, dass der Impfstoff beim Fressen des Köders in der Maulhöhle freigegeben und über die oropharyngeale Schleimhaut aufgenommen wird.

Als Köder können z.B. Hühnerköpfe oder Hackfleisch verwendet werden.

## Beispiel 1

Es wird ein an Hundenierenzellen adaptierter SAD-Stamm des Tollwutvirus verwendet. Dieser wird an die Baby

Hamster Nierenzellinie $BHK_{21}$ adaptiert, indem man ihn in Rouxflaschen im BHK-Wachstumsmedium unter Zusatz von 5% Kälberserum und 3% fötalem Kälberserum bei 33°C und einem pH zwischen 6,8 bis 7,4 bebrütet und in Abständen von 2 bis 3 Tagen passagiert. Die Inokulation der Zellen erfolgt dabei in Suspension bei einer Zelldichte von 0,5 bis $2 \times 10^5$ Zellen pro ml Medium. Nach ca. 48 Stunden ist der Zellrasen vollständig geschlossen und wird mit einer Trypsin-Versenlösung abgelöst und entsprechend der erhöhten Zellzahl ca. dreifach verdünnt und wiederum ausgesät. Nach weiteren 48 bis 72 Stunden erfolgt die Virusernte aus dem Zellüberstand. Weitere Resultete dieser Versuche sind in Tabelle 1 zusammengestellt.

Die Virustitration erfolgt auf Deckglaskulturen von $BHK_{21}$-Zellen im Multiwell-dish der Firma Costar. Die Costarplatte hat 24 Vertiefungen mit flachem Boden, einen Durchmesser von 16 mm und eine Tiefe von 18 mm.

Dem im BHK-Medium suspendierten Virus wird 10% Eigelb als Stabilisator beigemischt und jeweils 2 ml des Produkts in eine Durchdrückpackung mit einer Schüsselfolie aus einem 250 µ dicken, starren PVC-Film aus weichmacherfreiem Hart-PVC mit einem VC-Gehalt $<$ 1 ppm, und einer Deckblattfolie aus 40 µ dicker, auf der Innenseite thermolackierter (Vinylbasis 5 bis 6 $g/m^2$) Aluminiumfolie luftdicht abgefüllt und verschlossen.

Diese für Feldversuche vorgesehene Impfvirusmenge von ca. $10^7$ I.E. pro 2 ml werden in Hühnerköpfen fixiert und in dieser Form als Köder ausgelegt.

Tabelle 1

Zellzahl, prozentualer Anteil infizierter Zellen und Virusausbeute

| Beispiel | Inokulationskonzentration inf. Einheiten/ eingesäte Zelle | Versuchsbeginn Zellzahl/ml Suspension | nach 48 Stunden (beim Transfer) | | | | nach 96 Stunden ✗ | |
|---|---|---|---|---|---|---|---|---|
| | | | Zellzahl/ ml | Grad des CPE | % FA-positive Zellen | Virustiter inf.E./0.4 ml | Grad des CPE | Virustiter inf.E./0.4 ml |
| 2 | 0,009 | $3 \times 10^5$ | $5,4 \times 10^5$ | + | 100 | $10^{7.0}$ | ++/+++ | $10^{6.2}$ |
| 3 | 0,0045 | $3 \times 10^5$ | $3,1 \times 10^5$ | ± | 95 | $10^{7.2}$ | ++ | $10^{6.4}$ |
| 4 | 0,0009 | $3 \times 10^5$ | $6,1 \times 10^5$ | - | 90 | $10^{6.5}$ | +/++ | $10^{6.8}$ |
| 5 | 0,00045 | $3 \times 10^5$ | $3,2 \times 10^5$ | - | 80 | $10^{6.2}$ | +/++ | $10^{7.2}$ |

CPE = cytopathischer Effekt

✗ nach 96 Stunden in allen Kulturansätzen 100% der Zellen infiziert, d.h.FA-positiv.

## Patentansprüche

1. Verfahren zur Herstellung des neuen Virusstammes SAD produziert auf $BHK_{21}$, dadurch gekennzeichnet, dass man einen SAD-Stamm des Tollwutvirus an die Baby Hamster Nierenzellinie $BHK_{21}$ adaptiert, indem man den SAD-Stamm im BHK-Wachstumsmedium in einer Zellzahl von 0,5 - 4 x $10^5$ Zellen pro ml Medium unter Zusatz von Serum bei 33°C bis 37°C und einem pH von 6,8 bis 7,4 bebrütet und gegebenenfalls in Abständen von 2 bis 3 Tagen passagiert und gegebenenfalls gegen Wärmeinaktivierung stabilisiert.

2. Verfahren nach Patentanspruch 1, dadurch gekennzeichnet, dass man mit einer Zellzahl von 2 x $10^5$ Zellen/ml Medium in 5% Kälberserum und 3% fötalem Kälberserum bei 33 °C und einem pH von 7,4 bebrütet und nach 48 Stunden passagiert.

3. Verfahren nach Anspruch 1 oder 2, dadurch gekennzeichnet, dass als Stabilisator 5 bis 10% Eigelb oder Eipulver eingesetzt wird.

4. Virusstamm SAD produziert auf $BHK_{21}$.

5. Behälter zur oralen Verabreichung eines Impfstoffes gegen Tollwut an Füchsen, gekennzeichnet durch ein für Füchse zerbeissbares und kaubares Material, das den darin befindlichen Impfstoff in der Maulhöhle freigibt und ihn vor milieubedingtem Titerverlust schützt.

6. Behälter nach Anspruch 5, dadurch gekennzeichnet, dass er aus einer Schüsselfolie aus weichmacherfreiem Hart-PVC und einer Deckblattfolie aus auf der Innenseite auf Vinylbasis thermolackiertem Aluminium besteht.

7. Behälter nach Anspruch 6, dadurch gekennzeichnet, dass die Schüsselfolie 250 µ und die Aluminiumfolie 40 µ dick ist.

8. Mittel zur oralen Immunisierung von Füchsen gegen Tollwut, gekennzeichnet durch einen Behälter nach einem der Ansprüche 5 bis 7, in dem die zur Immunisierung notwendige Menge des nach einem der Patentansprüche 1 oder 2 hergestellten, gegebenenfalls stabilisierten Impfstoffes abgefüllt und luftdicht verschlossen ist.

9. Mittel nach Anspruch 8, gekennzeichnet durch eine notwendige Menge an gegebenenfalls stabilisiertem Impfstoff von 1,0 bis 2 ml, vorzugsweise 1,8 bis 2 ml.

10. Verfahren zur oralen Immunisierung von Füchsen gegen Tollwut, dadurch gekennzeichnet, dass man Mittel nach Anspruch 8 oder 9 in einem als Vehikel geeigneten Köder so fixiert, dass der Impfstoff beim Kauen und Zerbeissen des Köders in der Maulhöhle freigegeben und über die oropharyngeale Schleimhaut aufgenommen wird.

E